Europäisches Patentamt

European Patent Office

Office européen des brevets

(19)

(11) Veröffentlichungsnummer: **0 098 473**
**B1**

(12) **EUROPÄISCHE PATENTSCHRIFT**

(45) Veröffentlichungstag der Patentschrift:
07.12.88

(51) Int. Cl.⁴: **C 12 P 19/04, C 12 P 19/08**

(21) Anmeldenummer: **83106159.3**

(22) Anmeldetag: **23.06.83**

(54) **Verbessertes Verfahren zur Herstellung von exozellulären Biopolymeren.**

(30) Priorität: **01.07.82 DE 3224547**

(43) Veröffentlichungstag der Anmeldung:
**18.01.84 Patentblatt 84/3**

(45) Bekanntmachung des Hinweises auf die Patenterteilung:
**07.12.88 Patentblatt 88/49**

(84) Benannte Vertragsstaaten:
**AT BE CH DE FR GB IT LI LU NL SE**

(56) Entgegenhaltungen:
**EP-A- 0 058 364
EP-A- 0 074 775
WO-A-82/01563
GB-A- 1 141 107
GB-A- 1 174 322
US-A- 3 723 255
US-A- 3 997 398**

**CHEMICAL ABSTRACTS, Band 96, Nr. 1, Januar 1982,
Seite 460, Nr. 4951r, Columbus, Ohio, US; & JP - A - 81
109 593 (AGENCY OF INDUSTRIAL SCIENCES AND
TECHNOLOGY TOKUYAMA SODA CO., LTD.) 31.08.1981**

(73) Patentinhaber: **Henkel Kommanditgesellschaft auf
Aktien, Postfach 1100 Henkelstrasse 67,
D-4000 Düsseldorf-Holthausen (DE)**

(72) Erfinder: **Schindler, Joachim, Dr., Am Eichelkamp 158,
D-4010 Hilden (DE)**
Erfinder: **Weiss, Albrecht, Dr., Amselweg 8,
D-4006 Erkrath (DE)**
Erfinder: **Bahn, Michael, Dr., Frans-Hals-Weg 19,
D-4010 Hilden (DE)**

**Beschreibung**

Gegenstand der älteren europäischen Patentanmeldung 0 058 464 ist ein Verfahren zur Herstellung von Xanthomonas-Biopolymeren durch aerobes Züchten von Mikroorganismen der Gattung Xanthomonas in einem wässrigen Nährmedium, wobei dieses Verfahren dadurch gekennzeichnet ist, dass man die Mikroorganismen in einer unter Fermentationsbedingungen stabilen Wasser-in-Öl-Emulsion (W/O-Emulsion) züchtet. Ein ähnliches Verfahren wird in der ebenfalls nicht veröffentlichten europäischen Patentanmeldung EP-A-74 775 beschrieben. Bei dem Verfahren nach den genannten älteren Anmeldungen werden Mikroorganismen der Gattung Xanthomonas in einem üblichen Nährmedium angezüchtet, welches vor Erreichung seiner Endviskosität in einer mit Wasser nicht mischbaren Flüssigkeit dispergiert und danach so weiterbehandelt wird, dass die Produktion des Biopolymeren in der dispersen Phase stattfindet. Durch die vergleichsweise niedere Viskosität der W/O-Emulsion ist es möglich, Sauerstoff und andere wichtige Substrate selbst bei hohem Xanthan-Gehalt und an sich hoher Viskosität der wässrigen Phase zuzugeben.

Gegenstand der vorliegenden Anmeldung ist die Übertragung dieses Zuchtverfahrens auf andere Mikroorganismen, welche ebenfalls extrazelluläre Stoffwechselprodukte erzeugen, die das Nährmedium stark verdicken.

Ausser Bakterien der Gattung Xanthomonas sind in der Fachliteratur eine grosse Anzahl an Mikroorganismen beschrieben, welche exozellulär hydrophile Kolloide bilden. Verwiesen sei beispielsweise auf die folgenden Übersichtsarbeiten und die darin zitierte Literatur: M.E. Slodki, M.C. Cadmus, Adv. Appl. Microb. 23, 19 (1978); P.A. Sandford, Adv. i. Carbohydrate Chem. Biochem. 265 (1979); C.J. Lawson, I.W. Sutherland, Economic Mikrobiol. 2, 327 (1973).

Wie Xanthan sind auch die von anderen Mikroorganismen exozellulär produzierten Polymeren Polysaccharide. Im folgenden seien einige Beispiele genannt:

«Dextran», Stoffwechselprodukt des Bakteriums Leuconostoc mesenteroides, «bakterielle Alginsäuren» (Azotobacter vinelandii), «Arthrobacter Polysaccharide» (A. viscosus), «Succinoglucan» (Alcaligenes faecalis) sowie die Stoffwechselprodukte von Hefen wie Hansenula capsulata.

Bei allen Produkten handelt es sich um Polysaccharide, welche je nach Herkunft Glucose, Mannose, Galaktose, Glucuronsäure, Mannuronsäure, Guluronsäure enthalten. Ferner treten Acetyl- und Pyruvat-Reste auf.

Bei vielen dieser Polysaccharide ist bekannt, aus welchen Zuckern sie aufgebaut sind. Es liegen jedoch nur wenige Untersuchungen über die Struktur vor. Bekannt sind jedoch regelmässig aufgebaute Heteropolysaccharide mit bis zu 4 Monosaccharid-Einheiten als sich wiederholende Grundstruktur. Andererseits wurde auch offensichtlich rein statistische Verteilung der zugrundeliegenden Zucker beobachtet, so z.B. bei den bakteriellen Alginaten. Gängig sind weiterhin Blockstrukturen mit Blöcken aus zwei bis sechs gleichen Zuckermolekülen. Ferner wurden auch Polysaccharide aus nur einem Zuckerrest beobachtet, z.B. das Dextran.

Die Biopolymeren weisen in wässriger Lösung starke Verdickungseigenschaften auf. Teilweise ist Thixotropie zu beobachten. Soweit sie bereits technisch hergestellt werden, finden sie breite Anwendungen, z.B. in der Medizin, auf dem Nahrungsmittelsektor sowie als Verdickungsmittel und Konsistenzgeber in kosmetischen Präparationen, aber auch in einer Vielzahl chemisch-technischer Produkte.

Die grosstechnische Herstellung der Biopolymeren unterliegt allerdings – gerade wegen der besonderen Eigenschaften der entstehenden Produkte – schwerwiegenden Einschränkungen. Die bei der Züchtung in der Fermentermasse anfallenden Biopolymeren entwickeln bereits hier ihre ausgeprägten Verdickungswirkungen, so dass schon bei niedrigen Produktkonzentrationen in der Fermentationsphase Störungen auftreten, die sich beispielsweise in der Gefährdung des Substrattransfers und der gleichmässigen Verteilung des Sauerstoffs im Fermentationsgemisch ausdrücken. Durch den Einsatz hoher Scheerkräfte während der Fermentation kann aufgrund des teilweise auftretenden thixotropen Verhaltens der entstehenden verdickten wässrigen Nährlösung diese Gefährdung des Prozesses noch für einen beschränkten Zeitraum unterdrückt werden. So wird die Fermentation in Reaktionsgefässen durchgeführt, die intensive Rührwerke, beispielsweise mehrere mehrblättrige Flachschaufelturbinen enthalten. Unter Einsatz beträchtlicher Rührenergien gelingt es, das Reaktionsgemisch für einen beschränkten Zeitraum noch in so fliessfähigem Zustand zu halten, dass beispielsweise der Sauerstofftransport gewährleistet ist. Gleichwohl sind auch unter diesen extremen technischen Bedingungen dem Verfahren enge Grenzen gesetzt.

Je nach verwendetem Mikroorganismus werden in der Literatur in Abhängigkeit vom Mikroorganismus Produktausbeuten von 2–10% oder knapp darüber, jeweils berechnet als Trockensubstanz und bezogen auf den Fermentationsansatz, angegeben. Im praktischen Verfahren werden jedoch im allgemeinen unbefriedigende Ausbeuten erreicht.

Aufgabe der Erfindung ist es, das in der europäischen Patentanmeldung 0 058 364 beschriebene Verfahren zur Herstellung von Xanthomonas-Biopolymeren auf die Herstellung anderer exozellulärer Biopolymerer zu übertragen. Somit sollte die in der Fermentationsstufe nachteilige, beim Endprodukt letztlich aber gerade gewünschte Verdickungswirkung der Biopolymeren in der Fermentationsstufe so gemildert beziehungsweise beseitigt werden, dass die Herstellung in vielfältiger Weise erleichtert wird. Eine Teilaufgabe der Erfindung liegt auch hier in der Senkung der Viskosität der Flüssigkeitsphase im Fermenter unter Fermentationsbedingungen bei gleichwohl hoher Produktausbeute an Biopolymeren. Die Erfindung will weiterhin ein Züchtungsmedium schaffen, das die Einstellung höherer Feststoffkonzentrationen in der wässrigen Fermenterphase erlaubt und gleichwohl mit üblichen technischen bzw. apparativen Mitteln verarbeitbar bleibt. Erfindungsgemäss soll die Möglichkeit geschaffen werden, die Gewinnung der Polymeren absatzweise oder auch kontinuierlich mit verringertem Energiebedarf durchzuführen und gegenüber dem Stand der Technik gleiche oder insbesondere auch verbesserte Ausbeuten am gewünschten Verfahrensprodukt zu ermöglichen.

Zur technischen Lösung dieser Aufgabe wird auch erfindungsgemäss wie im Verfahren der älteren Anmeldung 0 058 364 vorgeschlagen, dass man die Mikroorganismen in einer unter Fermentationsbedingungen stabilen Wasser-in-Öl-Emulsion züchtet, wobei allerdings die eingesetzten Mikroorganismen von denen der älteren Anmeldung verschieden sind. Ansonsten gelten die allgemeinen Anweisungen der älteren Anmeldung sinngemäss.

Gegenstand der Erfindung ist ein Verfahren zur Herstellung von Biopolymeren durch aerobes Züchten von Mikroorganismen in einem wässrigen Nährmedium, bei dem an die Mikroorganismen in einer unter Fermentationsbedingungen stabilen Wasser-in-Öl-Emulsion (W/O-Emulsion) züchtet, dadurch gekennzeichnet, dass man exozelluläre Biopolymere mit Verdickungswirkung für das wässrige Nährmedium bildende Mikroorganismenstämme züchtet, die sich von den folgenden Bakterien und Hefe Spezies bzw. Gattungen ableiten:

Bacillus spp.
Leuconostoc spp.
Streptococcus mutans
Streptococcus spp.
Azotobacter spp.
Rhizobium spp.
Escherichia coli
Klebsiella aerogenes
Azotobacter xylinum
Arthrobacter viscosus
Pseudomonas aeruginosa
Achromobacter spp.
Alcaligenes faecalis var. myxogenes
Agrobacterium spp.
Erwinia spp.
Sphaerotilus natans
Rhodotorula spp.
Pichia spp.
Pachysolen tannophilus
Lipomyces spp.
Hansenula capsulata
Hansenula holstii
Cryptococcus spp.

Der Gegenstand der zuvor erwähnten älteren europäischen Patentanmeldung 0 058 364 wird hier nicht beansprucht.

In den Emulsionen – die im folgenden der Einfachheit halber als W/O-Emulsionen bezeichnet werden – liegt die wässrige Fermentationsphase mit ihren darin ablaufenden mikrobiellen Wachstums- bzw. Stoffwechselvorgängen als fein verteilte, disperse Phase in einer homogenen Ölphase vor. In jedem einzelnen Tröpfchen der dispersen wässrigen Phase kann der mikrobielle Züchtungsschritt ablaufen, eine Versorgung des Einzeltröpfchens mit Sauerstoff ist durch die Belüftung der W/O-Emulsion mit Sauerstoff bzw. einem sauerstoffhaltigen Gas insbesondere Luft gewährleistet. Der Viskositätsanstieg im jeweiligen Einzeltröpfchen der wässrigen Nährlösung macht sich für das im Fermenter vorliegenden Gesamtgemisch jedoch nicht oder nicht wesentlich bemerkbar, da die Viskosität der Reaktionsmasse im Fermenter weitgehend durch die geschlossene Ölphase bestimmt wird.

Es ist keineswegs selbstverständlich, dass sowohl die Lebensfähigkeit der Mikroorganismen als auch die Stabilität der Emulsionen unter Fermentationsbedingungen erhalten werden können, wenn Xanthomonas durch andere gram-negative Bakterien, andere gram-positive Bakterien, Pilze oder Hefen ersetzt wird. Erfindungsgemäss kann damit die in der älteren Anmeldung beschriebene substantielle Senkung der Viskosität in der Fermenter-Flüssigkeit nach der neuen Lehre weitgehend unabhängig von der Morphologie des Mikroorganismus auch auf die Herstellung anderer Biopolymerer übertragen werden.

Als organische Flüssigphase sind im erfindungsgemässen Verfahren grundsätzlich alle mit Wasser nicht mischbaren Flüssigphasen geeignet, die gegenüber den eingesetzten Mikroorganismen nicht toxisch sind, unter Fermentationsbedingungen keiner unerwünschten Veränderung unterliegen – insbesondere also unter Fermentationsbedingungen gegenüber Sauerstoffeinwirkung inert oder im wesentlichen inert sind – und die Ausbildung der W/O-Emulsionen erlauben. Bevorzugt werden organische Flüssigphasen, die im Gleichgewicht mit einer wässrigen Phase unter Verfahrensbedingungen nur beschränkte Wassermengen aufnehmen. So kann es bevorzugt sein, mit organischen Flüssigphasen zu arbeiten, deren Wassergehalt nicht über 0,5 Gew.-% und insbesondere nicht über 0,1 Gew.-% liegt. Bevorzugt kann es weiterhin sein, solche organischen Flüssigphasen einzusetzen, die auf das entstehende Biopolymere keine oder keine wesentliche solvatisierende und/oder quellende Wirkung ausüben. Die Ölkomponente muss wenigstens bei Fermentationstemperatur als Flüssigphase vorliegen. Bevorzugt liegt ihr Schmelzpunkt nicht über 25 °C, insbesondere nicht über 20 °C und vorzugsweise nicht höher als 10 °C. Weiterhin ist es bevorzugt, dass die Ölphase unter Fermentationsbedingungen nicht oder nur beschränkt flüchtig ist. Wenn hier auch keine zwingende Bedingung vorliegt, so kann doch das Fermentationsverfahren, das ja beispielsweise mit einem durch das Fermentationsgemisch durchgeleiteten Luftstrom arbeitet, bedeutend vereinfacht werden. Auf der anderen Seite ist zu berücksichtigen, dass in bestimmten Ausführungsformen der Erfindung bei der Aufarbeitung des Verfahrensproduktes die teilweise oder vollständige Abtrennung der Ölphase wünschenswert ist, so dass hier auf die jeweiligen Gesamtbedingungen zugeschnitten im Einzelfall optimale Verhältnisse eingestellt werden können.

Es hat sich als zweckmässig erwiesen, die organische Phase so zu wählen, dass sie drucklos durch Erwärmen sterilisiert werden kann.

Durch einfache Vorversuche lässt sich jeweils ermitteln, ob die Grundbedingungen der toxikologischen Verträglichkeit der jeweils ausgewählten Ölphase mit dem Mikroorganismenwachstum gewährleistet ist. Tatsächlich ist offenbar in breitem Rahmen organischer Flüssigphasen diese Voraussetzung gegeben.

Bei der Ölphase kann es sich weiterhin bevorzugt um beliebige hydrophobe Flüssigkeiten handeln, die von der entstehenden polymeren wässrigen Phase hinreichend leicht getrennt werden können. Geeignet sind unsubstituierte und/oder substituierte flüssige Kohlenwasserstoffverbindungen. Diese Bestimmung umfasst sowohl aliphatische als auch aromatische Verbindungen. Aliphatische Verbindungen können geradkettig, verzweigt oder cyclisch sein. Es kann sich dabei um bestimmte einzelne Kohlenwasserstoffverbindungen oder um Mischungen verschiedener Verbindungen handeln. Geeignet sind beispielsweise Kohlenwasserstofffraktionen des Bereichs der Mineralöle, der Kerosine oder Naphthas, aber auch organische Kohlenwasserstoffe wie Benzol, Xylol oder Toluol. Besonders geeignet kann eine Ölphase auf Basis verzweigter Kohlenwasserstoffverbindungen, beispielsweise auf Isoparaffinbasis aufgebaut sein. Es sind aber nicht nur unsubstituierte, gegebenenfalls ungesättigte, insbesondere olefinisch ungesättigte Kohlenwasserstoffverbindungen geeignet, auch hinreichend wasserunlösliche Alkohole mit insbesondere 8 bis 20, vorzugsweise 8–12 C-Atomen, pflanzliche Öle, Esteralkohole, Polyoäther, oder andere Heteroatome enthaltende Verbindungen, beispielsweise Siliconöle sind geeignet. Ein unter dem Handelsnamen «Isopar M» vertriebenes Isoparaffingemisch bzw. ein partielles Neutralisationsprodukt aus wasserunlöslichen Isoparaffinsäuren, die mit hydroxibenzyl-dialiphatischen Aminen teilneutralisiert worden sind und im einzelnen in der US-PS 2 262 720 beschrieben sind, hat sich als brauchbar erwiesen.

Das wässrige Fermentationsmedium kann unter den in der Literatur für den jeweiligen Mikroorganismus beschriebenen ausgewählt werden. Geeignete wässrige Fermentationsmedien sind beispielsweise beschrieben bei: J.R. Norris, D.W. Ribbons (Herausg.): Methods in Microbiology, Vol. 3A, Academic Press London (1970), oder bei R.L. Whistler, B.N. Bemiller (Herausg.): Industrial Gums, Polysaccharids and Derivativs (1973) im gleichen Verlag.

Typische wässrige Nährlösungen enthalten beispielsweise eine Quelle für organischen Stickstoff wie Maisquellwasser und/oder Sojamehl, Phosphatsalze wie Dialkalihydrogenphosphat und/oder Diammoniumhydrogenphosphat neben geeigneten Spurenelementen, insbesondere Magnesium und gegebenenfalls Mangan, Molybdän, Eisen und Calcium bei einem pH-Wert oberhalb 6, vorzugsweise oberhalb von 6,5 bis etwa 7. Die Nährlösung enthält zusätzlich ein geeignetes Kohlenhydrat zweckmässigerweise in der wässrigen Phase gelöst. Geeignete Kohlenhydrate sind z.B. Glucose, Saccharose, Maltose, Fructose, Lactose, bearbeitete, invertierte Zuckerrüben-Molassen, Invertzucker, filtrierte verdünnte Qualitätsstärke oder Gemische dieser Kohlenhydrate. Glucose ist eine bevorzugte Quelle des assimilierbaren Kohlenstoffs. Die Konzentration der assimilierbaren Kohlenhydratverbindung liegt üblicherweise im Bereich von 0,5–5 Gew.-%, bezogen auf die wässrige Phase. Die Verwendung höherer Konzentrationen an assimilierbaren Kohlenhydratverbindungen kann zur Ansammlung toxischer Nebenprodukte und damit zu einer Hemmung des Wachstums der Mikroorganismen führen. Auch können niedermolekulare, für viele Verwendungszwecke nicht geeignete, Stoffwechselprodukte entstehen. In manchen Fällen wird sogar eine vorzeitige Beendigung der Fermentation ausgelöst.

Möglich ist jedoch die Zugabe der assimilierbaren Kohlenhydratverbindung absatzweise oder kontinuierlich während des Fermentationsverlaufs, so dass letztlich beträchtlich höhere Glucosekonzentrationen umgesetzt werden können.

Eine besondere Ausführungsform der Erfindung besteht darin, n-Paraffine mit mehr als 10 C-Atomen als Ölphase zu verwenden und gleichzeitig Mikroorganismen zu züchten, welche diese als Quelle assimilierbaren Kohlenstoffs verwerten können. Derartige Mikroorganismen sind aus den Gattungen Corynebacterium, Brevibacterium und Mycobacterium bekannt, z.B. C. viscosum oder M. Lacticolum.

Eine weitere besondere Ausführungsform besteht in der Verwendung des Bacteriums Pseudomonas hydrogenovora, welches in Gegenwart von $H_2$- und $O_2$-Gas $CO_2$ als Quelle assimilierbaren Kohlenstoffs verwertet.

In allen Fällen liegt die Inkubationstemperatur zweckmässigerweise im Bereich von etwa 30 °C, beispielsweise bei 30 $\pm$ 10 °C. Die Fermentation kann bis zu einem Zeitraum von etwa 100 Stunden oder auch länger durchgeführt werden.

Bezüglich der in der Praxis üblicherweise eingesetzten Verfahren sei auf die in den eingangs zitierten Übersichtsarbeiten genannte Literatur verwiesen. Im allgemeinen wird wie bei der Xanthangewinnung gearbeitet. Ein in der Praxis üblicherweise eingesetztes Verfahren zur Herstellung von Xanthan und die dabei eingesetzten Reaktionshilfsmittel, insbesondere Nährlösungen und Inkubationsbedingungen, sind beispielsweise in der US-PS 3 236 831 beschrieben.

Die Auswahl und Abstimmung der geeigneten Ölphasen und Nährlösungen wird einerseits durch die erfindungsgemäss gewünschte optimale Erleichterung der Züchtungsstufe bestimmt, andererseits kann jedoch auch die beabsichtigte Verwendung der Produkte in die Auswahl insbesondere der Ölphase eingehen. Soll beispielsweise das Biopolymer auf dem Gebiet der sekundären bzw. tertiären Ausbeutung von Öllagerstätten eingesetzt werden, dann können unbedenklich Kohlenwasserstoffverbindungen als Ölphase Verwendung finden, die ernährungsphysiologisch weniger geeignet sein könnten. Ist daran gedacht, das Biopolymere dem Nahrungsmittelsektor zuzuführen, so kann es bevorzugt sein, mit einer Ölphase zu arbeiten, die aus sich heraus keine ernährungsphysiologischen Bedenken auslöst.

Zur Ausbildung der W/O-Emulsionen kann es bevorzugt sein, Emulgierhilfsmittel einzusetzen. Emulgiermittel vom Wasser-in-Öl-Typ (W/O-Emulgatoren) sind in der chemisch-technischen Praxis vielfach beschrieben. Die Emulgatoren lösen sich vorzugsweise in der Ölphase und führen bei der Verarbeitung mit einer wässrigen Phase zur Ausbildung des angestrebten W/O-Typs. Geeignete Verbindungen sind beispielsweise Sorbitanmonooleat, Sorbitanmonostearat, Hexadecylnatriumphthalat, Cetyl-stearylnatriumphthalat und weitere Verbindungen, wie sie in der DE-AS 1 089 173 genannt sind. Geeignet sind weiterhin W/O-Emulgatorsysteme, wie sie beispielsweise von der Firma Atlas Chemie GmbH unter den Handelsbezeichnungen «ARLACEL- oder SPAN-Produkte» vertrieben werden, wobei es sich um Verbindungen aus der Gruppe der Oleate mit einem geringen Anteil an «TWEEN»-Emulgatoren handelt. Weitere geeignete W/O-Emulgatorsysteme sind von der genannten Firma Atlas Chemie GmbH beschrieben und werden in der Literatur referiert als «Atlas-HLB-Systeme», vergleiche hierzu beispielsweise US-PS 3 996 180 und das einschlägige Prospektmaterial der genannten Firma. Hierzu gehören insbesondere Fettsäureester des Sorbitans, die mit bis zu 8 Mol Ethylenoxid umgesetzt worden sind. Zahlreiche W/O-Emulgatoren sind weiterhin beschrieben in Detergents and Emulsifier's Annual, John W. McCutcheon, Inc. Morristown N.J.

Die Auswahl der jeweils geeigneten Emulgierhilfsmittel kann wiederum durch die angestrebte Verfahrensführung und/oder die angestrebte Beschaffenheit des Verfahrensendproduktes bestimmt sein. So kann es im Rahmen des erfindungsgemässen Handelns liegen, soweit stabilisierte W/O-Emulsionen herzustellen, dass nach Verfahrensabschluss eine vollständige Trennung der Ölphase von der viskosen wässrigen Phase nur mit Schwierigkeiten verbunden wäre.

Auf der anderen Seite kann es beispielsweise eine im folgenden noch zu schildernde Verfahrensmodifikation wünschenswert machen, dass eine relativ einfache Trennung zwischen Ölphase und wässriger Phase eintritt. Dieser Fall kann beispielsweise gegeben sein, wenn entweder das Reaktionsprodukt als ganzes oder ein aus dem Fermenter abgezogener Teilstrom der W/O-Emulsion in beide Phasen getrennt und gegebenenfalls ein Anteil des abgetrennten Produktes wieder in den Fermenter zurückgeleitet werden soll. Für diesen zuletzt genannten Fall kann sich die Erfindung die Tatsache zunutze machen, dass auch im erfindungsgemässen Verfahren in der bevorzugten Ausführungsform im Fermenter unter ständiger Vermischung des Reaktionsgutes gearbeitet wird, so dass also unter den Arbeitsbedingungen der Fermentation die Einstellung der W/O-Emulsion gewährleistet ist, bei Ruhigstellung des Reaktionsgemisches jedoch eine hinreichend leichte Trennung der Phasen ermöglicht wird. Je nach den Besonderheiten des Einzelfalles wird die Auswahl der W/O-Emulgatoren vorgenommen.

Gegebenenfalls können im Verfahren der Erfindung weitere Hilfsmittel im Fermentationsansatz mitverwendet werden. Ein Beispiel hierfür sind Dispergierhilfsmittel.

Bevorzugte Mischungsverhältnisse von Ölphase zu der wässrigen Nährlösung liegen im Bereich von 15 bis 90 Gewichtsanteile Ölphase auf 85 bis 10 Gewichtsanteile wässriger Nährlösung bei Reaktionsbeginn. Insbesondere macht die Ölphase wenigstens 20 Gew.-% des W/O-Gemisches aus, wobei Ölmengen im Bereich von 20 bis 60 Gew.-% besonders bevorzugt sein können. Als im allgemeinen zweckmässig haben sich Ölmengen im Bereich von etwa 25 bis 50 Gew.-% – bezogen auf die W/O-Mischung – erwiesen.

Werden W/O-Emulgatoren mitverwendet, so liegt ihre Menge häufig im Bereich von etwa 0,1 bis 10 Gew.-% bezogen auf das Gesamtgemisch von W/O-Phase. Der bevorzugte Bereich für den Emulgatorgehalt liegt hier bei etwa 1 bis 5 Gew.-%. Es kann dabei weiterhin bevorzugt sein, dass der Emulgatorgehalt – bezogen auf die Ölphase – 3 bis 20 Gew.-% insbesondere etwa 5 bis 15 Gew.-% ausmacht. Die im Einzelfall gewählte Menge des Emulgierhilfsmittels kann dabei – in Abstimmung mit seinen durch die chemische Konstitution bestimmten Emulgiereigenschaften – bestimmt werden durch die zuvor geschilderte Absicht, stabile oder weniger stabile W/O-Emulsionen herzustellen.

Geeignete Mikroorganismen-Stämme, die exozelluläre Heteropolysaccharide bilden können, leiten sich von den folgenden Bakterien und Hefe-Spezies bzw. Gattungen ab:
Bakterien:
Bacillus spp.
Leuconostoc spp.
Streptococcus mutans
Streptococcus spp.
Azotobacter spp.
Rhizobium spp.
Escherichia coli
Klebsiella aerogenes
Azotobacter xylinum
Arthrobacter viscosus
Pseudomonas aeruginosa
Achromobacter spp.
Alcaligenes faecalis var. myxogenes
Agrobacterium spp.
Erwinia spp.
Sphaerotilus natans
Hefen:
Rhodotorula spp.
Pichia spp.
Pachysolen tannophilus
Lipomyces spp.
Hansenula capsulata
Hansenula holstii

Cryptococcus spp.
Torulopsis molischiana
Torulopsis pinus
Aureobasidium pullulans

Ein sauerstoffhaltiges Gas, insbesondere Luft, wird in den Produktionsfermenter mit herkömmlichen Mitteln eingeführt. Der Sauerstoffbedarf für die Fermentation kann den Gegebenheiten des Fermentationsverfahrens und des Sauerstofftransports angepasst werden, um die Ansammlung saurer toxischer Nebenprodukte herabzusetzen.

Gegenüber dem herkömmlichen Verfahren der Biopolymer-Produktion bringt die erfindungsgemässe Konzeption des Arbeitens in W/O-Emulsionen die Möglichkeit zu vielgestaltigen Abwandlungen unter Einstellung verbesserter Verfahrensbedingungen bzw. Ergebnisse. Im einzelnen gilt hier das folgende:

Die Anzüchtung der im grosstechnischen Verfahren eingesetzten Mikroorganismen kann mehrstufig in Nährmedien unterschiedlicher Zusammensetzung erfolgen, so wie es beispielsweise in der DE-OS 2 947 740 beschrieben und auf dem hier betroffenen Arbeitsgebiet ganz allgemein bekannt ist. Die so vorbereiteten Mikroorganismen werden dann in die für die Produktion vorgesehenen Nährlösungen gegeben und hier gezüchtet.

In einer Ausführungsform der Erfindung kann schon in dieser frühen Stufe die W/O-Emulsion ausgebildet werden, so dass – dargestellt anhand einer absatzweisen Verfahrensführung – bereits die Stufe der Vermehrung der Mikroorganismen in der erfindungsgemässen Emulsion erfolgt. Damit überlappend und anschliessend bildet sich das exozelluläre Stoffwechselprodukt der Mikroorganismenzüchtung.

In einer erfindungsgemäss bevorzugten Ausführungsform kann es allerdings zweckmässig sein, die Mikroorganismen zunächst im ölfreien wässrigen Nährmedium anzuzüchten, dabei die Vermehrung der Mikroorganismen in Abwesenheit der Ölphase zu einem nicht unbeträchtlichen Anteil voranzutreiben und erst dann die derart vorgezüchtete wässrige Fermentationslösung in die W/O-Emulsion zu überführen. In dieser Ausführungsform wird zweckmässigerweise so vorgegangen, dass die Emulgierung bzw. Dispergierung der wässrigen Phase in der Ölphase zu einem Zeitpunkt erfolgt, bei dem die sichere Verarbeitbarkeit der wässrigen Phase noch gewährleistet ist. So kann es in dieser Ausführungsform zweckmässig sein, dass man die ölfreie Anzüchtung in der wässrigen Phase bis zu Ausbeuten von wenigstens 0,1 Gew.-% Biopolymerem – vorzugsweise bis zu Ausbeuten von wenigstens 0,5 Gew.-% Biopolymerem – durchführt, bevor die Ausbildung der W/O-Emulsion vorgenommen wird. Die hier angegebenen Zahlenwerte für Gew.-% beziehen sich dabei jeweils auf die Trockensubstanz des Biopolymeren bezogen auf das Gewicht des wässrigen Fermentationsansatzes. Im einstufigen Verfahren ist es dabei möglich, die Vermehrungsstufe des Mikroorganismus zu wenigstens 30%, gegebenenfalls zu wenigstens 50% in der wässrigen Nährlösung ablaufen zu lassen, bevor die Überführung in die W/O-Emulsion erfolgen. Auch kann die Vermehrungsphase der Mikroorganismen vor Bildung der Emulsion und erst die Produktionsphase in W/O-Emulsion erfolgen. Durch einfache Vorversuche kann ein jeweils optimaler Zeitraum bestimmt werden, innerhalb dessen die sichere Überführung der wässrigen Phase in die disperse Aufteilung in der geschlossenen Ölphase unter den erfindungsgemässen Verfahrensbedingungen noch möglich ist.

Zur Ausbildung der W/O-Emulsion werden die angezüchtete Nährlösung und die Ölphase – in der zweckmässigerweise zuvor der gegebenenfalls mitverwendete Emulgator gelöst worden ist – miteinander vermischt und das Gemisch hinreichend intensiv mechanisch bewegt bzw. durchgearbeitet. In der bevorzugten Ausführungsform des erfindungsgemässen Verfahrens erfolgt die Züchtung auch innerhalb der W/O-Emulsion in Fermentern, die mit intensiv arbeitenden Mischelementen ausgerüstet sind. Die intensive Durchmischungswirkung solcher Rührelemente kann erfindungsgemäss dazu ausgenutzt werden, im Fermenter den Zustand der W/O-Emulsion aufrechtzuerhalten und sicherzustellen, und zwar selbst in solchen Fällen, in denen bei Ruhigstellung des Fermenterinhalts eine relativ einfache Phasenentmischung eintritt.

Der bewegte Fermenterinhalt wird in konventioneller Weise mit Sauerstoff bzw. mit einem sauerstoffhaltigen Gas, insbesondere Luft, begast. Der Sauerstofftransfer erfolgt über die Ölphase an jedes einzelne in disperser Phase emulgierte Teilchen der wässrigen, Mikroorganismen enthaltenden Nährlösung und ist dementsprechend auch in späten Stadien des Verfahrens weitaus weniger gestört als bei der bisher üblichen Verfahrenstechnik.

Erforderlichenfalls können wachstumsfördernde Bestandteile der Nährlösung absatzweise oder kontinuierlich nachgeliefert werden. So ist es etwa möglich, die assimilierbaren Kohlenstoff enthaltenden Verbindungen wie Glucose nach und nach dem Reaktionsgemisch zuzusetzen.

Eine gleichmässige Verteilung kann dadurch sichergestellt werden, dass diese Nährstoffe dem intensiv durchgearbeiteten Fermenter unmittelbar zugegeben werden. Entsprechendes gilt für andere Bestandteile der wässrigen Nährlösung, beispielsweise für Spurenelemente oder auch für die Zugabe von sonstigen benötigten Reaktanten, beispielsweise Basen, zur Regulierung des pH-Werts der dispersen wässrigen Phase.

Die Züchtung wird dann solange fortgeführt, bis die gewünschte Ausbeute eingestellt ist bzw. die Biopolymerbildung absinkt oder gar zum Stillstand kommt. Im absatzweisen Verfahren wird dann – je nach dem beabsichtigten Verwendungszweck des Produkts – in der Regel eine Trennung zwischen Ölphase und Biopolymer haltiger disperser Phase erfolgen, woraufhin das Polysaccharid in konventioneller Weise aus der wässrigen Phase abgetrennt und gereinigt werden kann.

Zum Brechen der W/O-Emulsionen können die in der chemischen Verfahrenspraxis üblichen Massnahmen eingesetzt werden, vergleiche hierzu beispielsweise Ullmann Enzyklopädie der technischen Chemie 1975, Band IV, Seite 453 und ff. sowie Houben-Weyl, Methoden der Organischen Chemie 1958, Band I/1, Seiten 219/220. Die Emulsionstrennung kann dementsprechend durch Zugabe emulsionsbrechender Substanzen, gegebenenfalls durch Einwirkung mechanischer Kräfte, wie Schütteln, Schlagen oder Druckeinwirkung, durch Temperaturerhöhung, durch Verdünnen oder Eindampfen der äusseren Phase und andere bekannte Massnahmen erfolgen. Insbesondere die Zerstörung von Emulsionen durch Zusatz geeigneter chemischer Stoffe, der sogenannten Desmulgatoren, ist in der Verfahrenstechnik bekannt. Der Handel hat eine Vielzahl von Emulsionsspaltern entwickelt, vergleiche hierzu beispielsweise Houben-Weyl aaO. Übliche Reinigungsschritte, beispielsweise das Auswaschen der Biopolymer haltigen wässrigen Phase mit geeigneten Lösungsmitteln, können sich anschliessen.

In einer besonderen Ausführungsform des erfindungsgemässen Verfahrens kann ein Teilstrom des Fermenterinhaltes absatzweise oder kontinuierlich abgezweigt, aufgearbeitet und gewünschtenfalls wenigstens anteilsweise wieder zurückgegeben werden. Über den abgezweigten Teilstrom kann beispielsweise die Zugabe von Reaktionshilfsmitteln oder aber auch die Zugabe frischer Mikroorganismenanteile erfolgen. Gleichzeitig kann über einen solchen abgezweigten Teilstrom eine absatzweise oder kontinuierliche Ausschleusung von Verfahrensprodukt aus dem Fermenter erfolgen. Für die Erfindung eröffnet sich hiermit die Möglichkeit der kontinuierlichen Verfahrensführung, wobei insbesondere durch regelmässige Kontrolle der das Biopolymer enthaltenden wässrigen Phase die Steuerung des Verfahrensablaufs in gewünschter Richtung möglich ist. Gegenüber dem in der Praxis bisher üblichen Verfahrenstyp des absatzweise arbeitenden einstufigen Verfahrens erschliessen sich hier neue technische fortschrittliche Möglichkeiten.

Die Isolierung des Polysaccharids aus der wässrigen Phase erfolgt in an sich bekannter Weise, beispielsweise durch Fällung und Trocknung. Zunächst kann die wässrige Phase hinreichend – zum Beispiel auf Temperaturen über 100 °C – erhitzt und sofort wieder abgekühlt werden, um die vorhandenen Mikroorganismen abzutöten und gegebenenfalls die Viskosität des Biopolymeren zu verbessern. Das Biopolymer wird dann durch Fällung, beispielsweise mit Alkoholen, anschliessendes Filtrieren und Trocknen gewonnen. Waschstufen zur Reinigung des Produkts können in an sich bekannter Weise eingeschlossen sein.

Beispiel 1

A) Der Stamm Azotobacter vinelandii DSM 85 wurde im 2 l-Fermenter (1,5 l-Füllvolumen) in folgendem Nährmedium bei 26 °C aerob angezüchtet:

Lösung A:    1,00  %    Glucose
            0,01  %    $CaCl_2 \cdot 2\ H_2O$
            0,01  %    $MgSO_4 \cdot 7\ H_2O$

Lösung B:    0,07  %    $K_2HPO_4$
            0,01  %    $KH_2PO_4$
            0,0005%    $Na_2MoO_4 \cdot 2\ H_2O$

Lösung C:    0,0013%    $FeSO_4 \cdot 7\ H_2O$

Die Lösungen A, B und C wurden getrennt sterilisiert. Der pH-Wert der vereinigten Lösungen wurde nach der Sterilisation auf pH 6,6 eingestellt.

Der Fermenter wurde mit 10 Vol.% einer 72 Std. alten Vorkultur beimpft. Durch kontinuierliche Zugabe weiterer Glucoselösung wurde die Zuckerkonzentration während des Fermentationsverlaufs ständig auf 1% gehalten. Nach 168 Std. Inkubation wurden in der Kulturlösung Viskositäten von 1400 mPas erreicht. Die Ausbeute an Polysaccharid betrug 16 g/l.

B) Die Fermentation unter W/O-Bedingungen wurde wie folgt durchgeführt: Die Anzucht des Stammes erfolgte zunächst unter den oben angeführten Bedingungen. Nach 120 Std. Inkubation wurden durch Einrühren von 30% Isopar M plus 1% Span 80 eine W/O-Emulsion im Fermenter erstellt. Die Viskosität der Kulturlösung konnte auf diese Weise deutlich gesenkt werden. Nach 192 Std. Kulturdauer betrug die Viskosität nur 75 mPas. Die Konzentration an präzipitiertem Polysaccharid betrug 20,5 g/l.

«Isopar M» ist ein isoparaffinisches Lösungsmittel der Esso Chemie mit folgenden Kennzahlen:

| | |
|---|---|
| Dichte bei 12 °C | 0,786 g/ml |
| Viskosität bei 25 °C | 2,46 mPas |
| Siedebereich | 204–247 °C |
| Aromatengehalt | 0,3 Gew.-% |
| Flammpunkt | 75 °C |

«Span 80» ist Sorbitanmonooleat, W/O-Emulgator mit HLB-Wert 4,3. Hersteller Atlas Chemie.

Beispiel 2

A) Der Stamm Leuconostoc mesenteroides DSM 20 240 wurde im 2 l-Fermenter (1,5 l Füllvolumen) bei 26 °C aerob in folgendem Nährmedium angezüchtet:
   1,00% Saccharose (getrennt sterilisiert)
   0,25% Hefeextrakt
   0,20% Maisquellwasser
   0,08% $K_2HPO_4$
   0,04% $NaH_2PO_4$
   0,05% $NH_4Cl$
   0,02% $MgSO_4 \cdot 7\ H_2O$
   0,01% $CaCl_2 \cdot 2\ H_2O$
       pH 6,5

Der Fermenter wurde mit 10 Vol.% einer 48 Std. alten Vorkultur beimpft. Die Saccharosekonzentration wurde über den gesamten Fermentationsverlauf durch kontinuierliche Zudosierung auf 1% gehalten. Nach 192 Std. Kulturdauer wurden in der Kulturlösung Viskositäten von 2750 mPas erreicht. Die gravimetrische Bestimmung des Dextrangehalts ergab 59 g/l.

B) Die Fermentation im W/O-Emulsionssystem wurde unter den gleichen Kulturbedingungen durchgeführt wie oben angegeben. Nach 120 Std. Kulturdauer wurde durch Einrühren von 30% Isopar M und 1% Span 80 in den Fermenter das W/O-System erstellt und die Viskosität in der Kulturlösung deutlich gesenkt. Die Fermentation wurde weitergeführt und die Kultur nach 196 Std. geerntet. Die Viskosität betrug 200 mPas und der Dextrangehalt 68 g/l.

## Patentansprüche

1. Verfahren zur Herstellung von Biopolymeren durch aerobes Züchten von Mikroorganismen in einem wässrigen Nährmedium, bei dem man die Mikroorganismen in einer unter Fermentationsbedingungen stabilen Wasser-in-Öl-Emulsion (W/O-Emulsion) züchtet, dadurch gekennzeichnet, dass man exozelluläre Biopolymere mit Verdickungswirkung für das wässrige Nährmedium bildende Mikroorganismenstämme züchtet, die sich von den folgenden Bakterien und Hefe Spezies bzw. Gattungen ableiten:

    Bacillus spp.
    Leuconostoc spp.
    Streptococcus mutans
    Streptococcus spp.
    Azotobacter spp.
    Rhizobium spp.
    Escherichia coli
    Klebsiella aerogenes
    Azotobacter xylinum
    Arthrobacter viscosus
    Pseudomonas aeruginosa
    Achromobacter spp.
    Alcaligenes faecalis var. myxogenes
    Agrobacterium spp.
    Erwinia spp.
    Sphaerotilus natans
    Rhodotorula spp.
    Pichia spp.
    Pachysolen tannophilus
    Lipomyces spp.
    Hansenula capsulata
    Hansenula holstii
    Cryptococcus spp.
    Torulopsis molischiana
    Torulopsis pinus
    Aureobasidium pullulans

2. Verfahren nach Anspruch 1, dadurch gekennzeichnet, dass man die Vermehrungsphase der Mikroorganismen beim Anzüchten wenigstens weitgehend in Abwesenheit der Ölphase durchführt und erst dann die Wasser-in-Öl-Emulsion bildet.

## Claims

1. A process for the production of biopolymers by aerobic culturing of microorganisms in an aqueous nutrient medium, in which the microorganisms are cultured in a water-in-oil emulsion (W/O emulsion) stable under fermentation conditions, characterized in that exocellular biopolymers having a thickening effect on microorganism strains which form the aqueous nutrient medium and which are derived from the following bacteria and yeast species or geni are cultured:

    Bacillus spp.
    Leuconostoc spp.
    Streptococcus mutans
    Streptococcus spp.
    Azotobacter spp.
    Rhizobium spp.
    Escherichia coli
    Klebsiella aerogenes
    Azotobacter xylinum
    Arthrobacter viscosus
    Pseudomonas aeruginosa
    Achromobacter spp.
    Alcaligenes faecalis var. myxogenes
    Agrobacterium spp.
    Erwinia spp.
    Sphaerotilus natans
    Rhodotorula spp.
    Pichia spp.
    Pachysolen tannophilus
    Lipomyces spp.
    Hansenula capsulata
    Hansenula holstii
    Cryptococcus spp.
    Torulopsis molischiana
    Torulopsis pinus
    Aureobasidium pullulans.

2. A process as claimed in claim 1, characterized in that the microorganism propagation phase is carried out at least substantially in the absence of the oil phase and is followed by formation of the water-in-oil emulsion.

## Revendications

1. Procédé de préparation de biopolymères par culture aérobie de microorganismes dans un milieu nutritif aqueux, le microorganisme étant cultivé dans une émulsion d'eau dans l'huile (émulsion eau/huile) stable dans les conditions de fermentation, caractérisé en ce qu'on cultive des souches de microorganismes formant des biopolymères exocellulaires avec une action épaississante sur le milieu nutritif aqueux, qui proviennent des genres et espèces de bactéries et levures qui suivent:

    Bacillus spp.
    Leuconostoc spp.
    Streptococcus mutans
    Streptococcus spp.
    Azotobacter spp.
    Rhizobium spp.
    Escherichia coli
    Klebsiella aerogenes
    Azotobacter xylinum
    Arthrobacter viscosus
    Pseudomonas aeruginosa
    Achromobacter spp.
    Alcaligenes faecalis var. myxogenes
    Agrobacterium spp.
    Erwinia spp.
    Sphaerotilus natans

Rhodotorula spp.
Pichia spp.
Pachysolen tannophilus
Lipomyces spp.
Hansenula capsulata
Hansenula holstii
Cryptococcus spp.
Torulopsis molischiana

Torulopsis pinus
Aureobasidium pullulans

2. Procédé suivant la revendication 1, caractérisé en ce qu'on forme la phase de multiplication des microorganismes lors de la culture au moins pour une grande part en l'absence de phase huileuse et que l'on forme seulement après l'émulsion d'eau dans l'huile.